# EUROPÄISCHE PATENTANMELDUNG

(11) **EP 1 591 104 A1**
(43) Veröffentlichungstag der Anmeldung: **02.11.2005**
(21) Anmeldenummer: 04006336.4
(22) Anmeldetag: 17.03.2004
(51) Int. Cl.: A61K 7/42

(54) **Verwendung von Antioxidantien zur Herstellung einer pharmazeutischen oder kosmetischen Zusammensetzung zum Schutz der Haut gegen Schädigung durch Infrarot-Strahlung**

(71) Anmelder: STADA ARZNEIMITTEL AG, 61118 Bad Vilbel (DE)
(72) Erfinder:
(74) Vertreter: Hamm, Volker

(57) **Zusammenfassung**

Die Erfindung betrifft die Verwendung von Antioxidantien zur Herstellung einer pharmazeutischen oder kosmetischen Zusammensetzung zum Schutz der Haut gegen Schädigung durch Infrarot-Strahlung.

## Beschreibung

Die Erfindung betrifft die Verwendung von Antioxidantien zur Herstellung einer pharmazeutischen oder kosmetischen Zusammensetzung zum Schutz der Haut gegen Schädigung durch Infrarot-Strahlung.

Die schädigende Wirkung des ultravioletten Teils der Sonnenstrahlung auf die Haut ist seit langem bekannt. Zum Schutz der Haut sowohl vor UVA-Strahlung als auch vor UVB-Strahlung sind zahlreiche chemische und physikalische Filtersubstanzen entwickelt worden, die durch Auftragen auf die Haut in Form dermatologischer oder kosmetischer Zusammensetzungen die Wirkung der Sonnenstrahlen im UVA- und UVB-Bereich abschwächen.

Neuere Untersuchungen haben ergeben, dass auch infrarote Strahlung einen schädigenden Einfluss auf die Haut hat. Infrarot-Strahlung ist nicht nur wesentlicher Bestandteil des Sonnenlichts, sondern auch des künstlichen Lichts.

Nahes Infrarotlicht, IR-A-Licht, mit einem Wellenlängenbereich von 760-1400 nm, aktiviert die Bildung von Matrixmetalloproteinase-1 in den Fibroblasten der menschlichen Haut, vermittelt über Aktivierung des MAPK-Signalwegs. Matrixmetalloproteinase-1 ist für die vorzeitige Hautalterung mitverantwortlich.

Der photobiologische Mechanismus, über den das IR-A-Licht die MAPKinase aktiviert, ist unbekannt.

Aufgabe der vorliegenden Erfindung ist es, eine Möglichkeit zum Schutz der Haut gegen Schädigung durch IR-Strahlung anzugeben.

Diese Aufgabe wird durch die im unabhängigen Anspruch definierte Verwendung gelöst.

Die abhängigen Ansprüche sind vorteilhafte Ausführungsformen der Erfindung.

Überraschend wurde gefunden, dass die Verwendung von Antioxidantien zur Herstellung von pharmazeutischen oder kosmetischen Zusammensetzungen zu Produkten führt, die die Haut wirksam gegen den schädigenden Einfluss von IR-Strahlung schützen.

Es hat sich überraschender Weise herausgestellt, dass pharmazeutische oder kosmetische Zusammensetzungen umfassend Antioxidantien ausgewählt aus der Gruppe bestehend aus: Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide (z.B. Lycopin, Lutein), Carotine (z.B. α-Carotin, β-Carotin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Lionleyl-, Cholesteryl- und Glycerylester) sowie deren Salze, Dilaurylthiodipropionat, Distearylthiodiproprionat, Thiodipropionsäure und deren Derivate (Ester, Ether, Peptide, Lipide, Nukleotide, Nukleoside und deren Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide (z.B. Rutin und dessen Derivate, z.B. Troxerutin), Polyphenole, Catechine, Ubichinon und Ubichinol und deren Derivate, Vitamin C und dessen Derivate (z.B. Ascorbylpalmitat, Mg-Ascorbylphosphat, Ascorbylacetat), Tocopherole und deren Derivate (z.B. Vitamin E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Liptide), Phytoen, D-Biotin, Coenzym Q10, α-Glycosylrutin, Carnitin, Carnosin, natürlich und/oder synthetische Isoflavonoide, Kreatin, Taurin und/oder β-Alanin sowie 8-Hexadecen-1,16-dicarbonsäure sowie Vitis Vinifera (Grape) seed extract, Haferextrakt, Cichorium intubybus (chicory) leaf extract, Leontopodium Alpinum Extract (Edelweiss), Grünteeextrakt, Curcumin, Silymarin, Apigenin, Resveratrol oder Emblica einen wirksamen Schutz vor IR-Strahlung bieten. Sie können einzeln oder in Kombination mehrerer Antioxidantien eingesetzt werden.

Emblica wird aus den Früchten der Heilpflanze Phyllantus emblica schonend durch Wasserdampfdestillation gewonnen. Hauptinhaltsstoffe von Emblica sind die niedermolekularen Gerbsäuren Emblicanin A und B.

Unabhängig von einer wissenschaftlichen Lehre vermutet die Anmelderin, dass der wirksame IR-Schutz durch Verabreichung von Antioxidantien enthaltenden pharmazeutischen Zusammensetzungen auf einem Einfluss der Antioxidantien auf die Aktivierung des MAPK-Signalwegs beruht. Der photobiologische Mechanismus, über den das IR-Licht die MAPKinase aktiviert, ist zwar unbekannt, doch vermutet die Anmelderin, dass diese Aktivierung über reaktive Sauerstoffspezies, sogenannte freie Radikale, vermittelt wird. Die erfindungsgemäße Verwendung von Antioxidantien führt vermutlich zu einer Minderung des Gehaltes an reaktiver Sauerstoffspezies. Dadurch wird der durch IR-Strahlung aktivierten und über den MAPKinase-Signalweg vermittelten Bildung von Matrixmetalloproteinase-1 entgegengewirkt.

Die erfindungsgemäßen Zusammensetzungen können topisch, oral, parenteral oder rektal verabreicht werden.

Die topisch zu verabreichenden Zusammensetzungen werden bevorzugt in Form von Creme, Salbe, Paste, Lotion, Gel, Hydrodispersionsgel, Aerosol, Spray, oder Stift auf die Haut aufgetragen.

Als bevorzugte Antioxidantien für die topische Anwendung haben sich insbesondere Vitamin C, Vitamin E, β-Carotin, Emblica, Lutein, Lycopen und Polyphenole sowie die entsprechenden Ester und Salze sowie Grünteeextrakt, Curcumin, Silymarin, Apigenin und Resveratrol erwiesen.

Der Gehalt an Antioxidantien in den topisch anzuwendenden pharmazeutischen Zusammensetzungen beträgt 0,001-25 Gew.-%, insbesondere 0,01-10 Gew.-%.

Topisch zu verabreichende Zusammensetzungen können weitere übliche Zusatzstoffe für dermatologische Zusammensetzungen, wie z.B. Verdickungsmittel, Glättungsmittel, anfeuchtende und/oder feuchthaltende Substanzen, oberflächenaktive Mittel, Konservierungsmittel, Entschäumer, Fette, Öle, Wachse, Konservierungsmittel, Bakterizide, Parfüme, Treibmittel, Farbstoffe und/oder Pigmente oder dergleichen umfassen.

Als Verdickungsmittel werden, sofern enthalten, bevorzugt Xanthan oder Polyacrylsäuren und deren Derivate, wie beispielsweise die aus der Carbopol-Reihe oder auch Pemulen TR-1 eingesetzt.

Derartige Zusammensetzungen werden bevorzugt vor, während oder nach einer Bestrahlung der Haut mit IR-Strahlen angewendet.

Die topisch zu verabreichenden Zusammensetzungen umfassen in bevorzugten Ausführungsformen der Erfindung des weiteren einen oder mehrere anorganische und/oder organische UV-Filter.

Derartige Zusammensetzungen können bevorzugt während der Bestrahlung der Haut mit Sonnenstrahlen zum Schutz vor Schäden durch IR- und UV-Strahlen angewendet werden.

Vorteilhafte anorganische UV-Filter sind Metalloxide insbesondere Oxide des Titans oder Zinks. Wenn anorganische Pigmente enthalten sind, werden diese vorzugsweise in mikronisierter Form eingesetzt, vorzugsweise in Teilchengrößen kleiner 100 µm. Sie sind dann bevorzugt in Mengen von 1-25 Gew.-%, insbesondere 1-10 Gew.-% enthalten. Dabei ist es besonders vorteilhaft, wenn die physikalischen Lichtschutzfilter in hydrophober Form vorliegen, das heißt, dass sie oberflächig wasserabweisend ausgestaltet sind. Dies kann z.B. dadurch erfolgen, dass die Pigmentteilchen mit einer hydrophoben Oberflächenschicht aus Silikon überzogen werden. Derartige Pigmente sind kommerziell erhältlich beispielsweise von der Firma Tayca unter der Handelsbezeichnung MT100T.

Erfindungsgemäße organische UV-Filter sind öllösliche oder wasserlösliche UV-A-und/oder UV-B-Filter.

Als geeignete öllösliche UV-Filter sind zu nennen:
- Ester der Zimtsäure, beispielsweise P-Methoxyzimtsäureisoamylester (Neo Heliopan 1000) oder Methoxyzimtsäureethylhexylester (Neo Heliopan AV);
- P-Aminobenzoesäure, ihre Ester und Derivate, beispielsweise 2-Ethylhexyl-p-dimethylaminobenzoat (Eskalol 507);
- Benzophenone, beispielsweise Benzophenon-3 (Neo Heliopan BB);
- Benzylidencampherderivate, beispielsweise 4-Methylbenzylidencampher (Eusolex 6300);
- Hydroxyphenyltriazine, beispielsweise Bis-ethylhexyloxyphenolmethoxyphenyl (Tinosorb S);
- Dibenzoylmethane, beispielsweise Butylmethoxydibenzoylmethan (Parsol 1789);
- Octyltriazon (Uvinul T150) und
- Octocrylen (Neo Heliopan 303).

Öllösliche UV-Filter sind, sofern enthalten, bevorzugt in Mengen von 0,1-15 Gew.-%, vorzugsweise 0,5-10 Gew.-%, insbesondere 0,5-8 Gew.-%, in den topisch zu verabreichenden Zusammensetzungen enthalten.

Als geeignete wasserlösliche UV-Filter sind zu nennen:
- Salze der 2-Phenylbenzimidazol-5-sulfonsäure (Neo Heliopan Hydro), sowie die Sulfonsäure selbst;
- 2,2'-(1,4-Phenylen)bis(1H-benzimidazol-4,6-disulfonsäure Mononatriumsalz) (Neo Heliopan AP)
- Sulfonsäure-Derivate des 3-Benzylidencamphers, beispielsweise 4-(2-oxo-3-bornylidenmethyl)benzolsulfonsäure, 2-Methyl-5-(2-oxo-3-bornyliden-methyl)sulfonsäure und ihre Salze.

Auch die wasserlöslichen UV-Filter sind, sofern enthalten, bevorzugt in Mengen von 0,1-15 Gew.-%, vorzugsweise 0,5-10 Gew.-%, insbesondere 0,5-8 Gew.-%, in den topisch zu verabreichenden Zusammensetzungen enthalten.

Die oral zu verabreichenden Zusammensetzungen werden bevorzugt in Form von Tabletten, Kapseln, Saft, Tropfen, Brausetabletten oder Lyophilisat verabreicht.

Sie können übliche Hilfsstoffe, wie z.B. Lösungsmittel, Lösungsbeschleuniger, Emulgatoren, Lösungsvermittler, Netzmittel, Antischaummittel, Salzbildner, Puffer, Gelbildner, Verdickungsmittel, Filmbildner, Bindemittel, Gleitmittel, Schmiermittel, Formtrennmittel, Fließregulierungsmittel, Zerfallsbeschleuniger oder Sprengmittel, Sorptionsmittel, Füllstoffe sowie Konservierungsmittel, Geschmacks- und Geruchskorrigenzien sowie Färbemittel enthalten.

Bevorzugte Antioxidantien für die orale Verabreichung sind insbesondere Vitamin E und dessen Derivate, Vitamin C und dessen Derivate, Beta-Carotin, Lutein, Lycopin, Rutin und dessen Derivate, wie Troxerutin sowie Emblica und Selen.

Der Gehalt an Antioxidantien in den oral zu verabreichenden pharmazeutischen Zusammensetzungen beträgt 1-600 mg, bevorzugt 2-300 mg, besonders bevorzugt 5-200 mg.

Die Antioxidantien können in der nach üblichen Verfahren ablaufenden Herstellung von kosmetischen und pharmazeutischen Zusammensetzungen verwendet werden.

Die folgenden Ausführungsbeispiele verdeutlichen die Erfindung, ohne sie zu beschränken.

### Beispiel 1 (Spray)

| | |
|---|---|
| Vergällter Alkohol | 81 g |
| Uvinul T150®(Ethylhexyl Triazone) | 5,0 g |
| Neo Heliopan 1000®(Isoamyl p-Methoxycinnamate) | 3,0 g |
| Neo Heliopan 303®(Octocrylene) | 3,0 g |
| Parsol 1789®(Butylmethoxydibenzoylmethane) | 0,5 g |
| α-Bisabolol nat. | 0,1 g |
| DL-α-Tocopherol | 1,0 g |
| E-Wasser | 6,3 g |
| β-Carotin | 0,1 g |

Ethylhexyl Triazone und Butyl Methoxydibenzoylmethane in Isoamyl p-Methoxycinnamate und Octocrylene lösen, β-Carotin darin lösen, Bisabolol, Tocopherol zugeben. Alkohol und Wasser zugeben, mischen.

### Beispiel 2 (Hydrodispersionsgel)

| | |
|---|---|
| Neo Heliopan 1000®(Isoamyl p-Methoxycinnamate) | 5,0 g |
| Neo Heliopan AV®(Ethylhexyl Methoxycinnamate) | 5,0 g |
| Eusolex 6300®(4-Methylbenzylidene Camphor) | 3,0 g |
| Parsol 1789®(Butyl Methoxydibenzoylmethane) | 2,0 g |
| Uvinul T150®(Ethylhexyl Triazone) | 2,0 g |
| α-Tocopherol | 0,5 g |
| Emblica | 0,5 g |
| Glycerin | 3,0 g |
| Disodium EDTA | 0,1 g |
| Panthenol | 1,0 g |
| E-Wasser | 68,35 g |
| Pemulen TR1®(Acrylates C10-30 Alkyl Acrylate Crosspolymer) | 0,4 g |
| Natriumhydroxid rein | 0,15 g |
| Vergällter Alkohol | 9,0 g |

### Phase A:

4-Methylbenzylidene Camphor, Ethylhexyl Triazone und Butyl Methoxydibenzoylmethane in Isoamyl p-Methoxycinnamate und Ethylhexyl Methoxycinnamate lösen. Tocopherol zugeben, Acrylates C 10-30 Alkyl Acrylate Crosspolymer dispergieren.

### Phase B:

Natriumhydroxid rein in einem Teil E-Wasser lösen

### Phase C:

Emblica, Glycerin Disodium EDTA und Panthenol in Wasser lösen

### Phase D:

### Vergällter Alkohol

Phase C zu Phase A geben, unter Homogenisieren mischen, Phase B zugeben, unter Rühren mischen. Phase D zugeben, unter Rühren mischen.

### Beispiel 3 (Lotion)

| | |
|---|---|
| Tego Care 450®(Polyglyceryl-3 Methylglucose Distearate) | 2,0 g |
| Softisan 100®(Hydrogenated Coco-Glycerides) | 0,5 g |
| Cutina CP®(Cetyl Palmitate) | 0,5 g |
| Lanette C®(Cetyl Alcohol) | 1,5 g |
| Myritol 331®(Cocoglycerides) | 5,0 g |
| Neo Heliopan 1000®(Isoamyl p-Methoxycinnamate) | 5,0 g |
| Uvinul T150 (Ethylhexyl Triazone) | 2,0 g |
| Eusolex 6300®(4-Methylbenzylidene Camphor) | 3,0 g |
| Parsol 1789®(Butylmethoxydibenzoylmethane) | 1,0 g |
| Carbopol 981®(Carbomer) | 0,2 g |
| α-Tocopherolacetat | 1,0 g |
| Lutein | 0,5 g |
| Glycerin | 4,0 g |
| Xanthan Gum | 0,1 g |
| E-Wasser | 64,654 g |
| Natriumhydroxid rein | 0,046 g |
| Vergällter Alkohol | 9,0 g |

### Phase A:

Polyglyceryl-3 Methylglucose Distearate, Hydrogenated Coco-Glycerides, Cetyl Palmitate, Cetyl Alcohol und Cocoglycerides erwärmen, darin Ethylhexyl Triazone, 4-Methylbenzylidene Camphor und Butyl Methoxydibenzoylmethane lösen. Tocopherolacetate und Lutein darin lösen.

### Phase B:

Natriumhydroxide in einem Teil Wasser lösen

### Phase C:

Glycerin und E-Wasser mischen, Xanthan Gum und Carbomer darin quellen

### Phase D:

### Vergällter Alcohol

Phase C zu Phase A geben, unter Homogenisieren mischen, Phase B zugeben, unter Homogenisieren mischen, Phase D zugeben, unter Homogenisieren mischen.

### Beispiel 4 (Hydrodispersionsgel)

| | |
|---|---|
| Eutanol G®(Octyldodecanol) | 5,0 g |
| Myritol 318®(Carpylic/Capric Triglyceride) | 5,0 g |
| Eusolex 6300®(4-Methylbenzylidene Camphor) | 3,0 g |
| Parsol 1789®(Butyl Methoxydibenzoylmethane) | 1,0 g |
| DL-α-Tocopherol | 0,5 g |
| Pemulen TR1®(Acrylates C10-30 Alkyl Acrylate Crosspolymer) | 0,4 g |
| Panthenol | 1,0 g |
| Glycerin | 3,0 g |
| Disodium EDTA | 0,1 g |
| E-Wasser | 58,85 g |
| Natriumhydroxid rein | 0,15 g |
| Uvinul T150®(Ethylhexyl Triazone) | 2,0 g |
| Vergällter Alkohol | 9,0 g |
| Isopropylmyristat | 5,0 g |
| Neo Heliopan AV (Ethylhexylmethoxycinnate) | 5,0 g |
| Edelweissextrakt | 1,0 g |

### Phase A:

4-Methylbenzyliden Camphor, Ethylhexyl Triazone und Butyl Methoxydibenzoylmethane in Ethylhexyl Methoxycinnamate und Caprylic/Capric Triglyceride lösen. Octyldodecanol, Isopropylmyristate und Tocopherol zugeben, Acrylates C 10-30 Alkyl Acrylate Crosspolymer dispergieren.

### Phase B:

Natriumhydroxid rein in einem Teil E-Wasser lösen

### Phase C:

Edelweissextrakt, Glycerin Disodium EDTA und Panthenol in Wasser lösen

### Phase D:

### Vergällter Alkohol

Phase C zu Phase A geben, unter Homogenisieren mischen, Phase B zugeben, unter Rühren mischen, Phase D zugeben, unter Rühren mischen.

### Beispiel 5 (W/O-Creme)

| Phase A: | |
|---|---|
| Dehymuls PGPH (Polygylceryl-2 Dipolyhydroxystearate) | 3,0 g |
| Monomuls 90-018 (Glyceryl Oleate) | 2,0 g |
| Myritol 318 (Caprylic/Capric Triglyceride) | 6,0 g |
| Isopropylmyristat | 10,0 g |
| α-Tocopherolacetat | 1,0 g |
| Bienenwachs | 1,0 g |
| Magnesiumstearat | 0,5 g |
| Isoamyl p-Methoxycinnamate | 10,0 g |
| 4-Methylbenzylidene Camphor | 4,0 g |
| Ethylhexyl Triazone | 1,0 g |

### Unter Erwärmen lösen

| Phase B: | |
|---|---|
| E-Wasser | 45,8 g |
| Disodium EDTA | 0,1 g |
| Pentylene Glycol | 3,0 g |
| Magnesiumsulfat | 0,5 g |
| α-Bisabolol | 0,1 g |
| Grünteeextrakt | 1,0 g |

### Unter Rühren lösen

| Phase C: | |
|---|---|
| Titaniumdioxide | 3,0 g |

| Phase D: | |
|---|---|
| Alcohol | 8,0 g |

Phase B zu Phase A geben, unter Homogenisieren mischen. Unter Homogenisieren Phase C zugeben. Phase D zugeben, unter Homogenisieren mischen.

### Beispiel 6 (Hydrodispersionsgel)

| Phase A: | |
|---|---|
| Neo Heliopan AV (Ethylhexyl Methoxycinnamate) | 2,5 g |
| Neo Heliopan 1000 (Isoamyl p-Methoxycinnamate) | 2,5 g |
| Neo Heliopan 303 (Octocrylene) | 2,5 g |
| α-Tocopherolacetat | 0,25 g |
| Acrylates C 10-30 Alkyl Acrylate Crosspolymer | 0,5 g |

### Mischen und dispergieren

| Phase B: | |
|---|---|
| E-Wasser | 30,0 g |
| Neo Heliopan Hydro (Phenylbenzimidazole Sulfonic Acid) | 2,0 g |
| Neo Heliopan AP (Disodium Phenyl Dibenzimidazole Tetrasulfonate) | 2,0 g |
| Natrium Hydroxide rein | 0,7 g |

### Lösung herstellen

| Phase C: | |
|---|---|
| E-Wasser | 44,0 g |
| Vergällter Alkohol | 9,5 g |
| Glycerin | 1,5 g |
| Panthenol | 0,5 g |
| Disodium EDTA | 0,05 g |
| Vitamin C | 0,5 g |
| Emblica | 1,0 g |

### Lösung herstellen

Phasen B und C mischen und unter Homogenisieren zu Phase A geben.

### Beispiel 7 (Hartkapsel zur oralen Verabreichung)

| | |
|---|---|
| Ascorbinsäure | 120 mg |
| Vitamin-E-acetat | 30 mg |
| Beta-Carotin | 15 mg |
| Selenhefe | 25 mg entsprechend 50 µg Selen |
| Gelatine | 75 mg aus Kapselhülle |

Die Bestandteile 1-4 werden entsprechend ihrem Mengenverhältnis abgewogen und gesiebt. Danach werden sie in einem Freifallmischer gemischt und die fertige Mischung auf einer geeigneten Kapselmaschine in Hart-Kapseln gefüllt.

### Beispiel 8 (Hartkapsel zur oralen Verabreichung)

| | |
|---|---|
| Lykopen | 8 mg |
| Beta-Carotin | 8 mg |
| Lutein | 8 mg |
| Vitamin C | 50 mg |
| Vitamin E | 25 mg |
| Lactose | 125 mg |
| Magnesiumstearat | 3 mg |
| Aerosil | 3 mg |
| Gelatine | 75 mg aus Kapselhülle |

Die Bestandteile 1-7 werden entsprechend ihrem Mengenverhältnis abgewogen und gesiebt. Danach werden sie in einem Freifallmischer gemischt. Magnesiumstearat wird abgewogen und gesiebt und zu der Hauptmischung aus 1-7 hinzugegeben und gemischt. Die fertige Mischung wird auf einer geeigneten Kapselmaschine in Hart-Kapseln gefüllt.

## Patentansprüche

1. Verwendung von Antioxidantien zur Herstellung einer pharmazeutischen oder kosmetischen Zusammensetzung zum Schutz der Haut gegen Infrarotstrahlung.

2. Verwendung nach Anspruch 1 zur Herstellung einer topisch zu verabreichenden Zusammensetzung.

3. Verwendung nach Anspruch 2,
**dadurch gekennzeichnet, dass** die Antioxidantien ausgewählt sind aus der Gruppe bestehend aus N-Acetylcystein, Vitamin E, β-Carotin, Emblica, Vitamin C-Derivate, Grünteeextrakt, Edelweissextrakt, Lutein, Lycopen, Traubenkernextrakt, Chicory-Extrakt, Curcumin, Silymarin, Apigenin, Resveratrol.

4. Verwendung nach Anspruch 2 oder 3,
**dadurch gekennzeichnet, dass** der Gehalt an Antioxidantien in der Zusammensetzung 0,001-25 Gew.-%, bevorzugt 0,01-10 Gew.-% beträgt.

5. Verwendung nach einem der Ansprüche 2 bis 4,
**dadurch gekennzeichnet, dass** die Zusammensetzung des weiteren einen oder mehrere UV-Filter umfasst.

6. Verwendung nach einem der Ansprüche 2 bis 5,
**dadurch gekennzeichnet, dass** die Zusammensetzung des weiteren Hilfsstoffe enthält, die unter Verdickungsmitteln, Glättungsmitteln, anfeuchtenden und/oder feuchthaltenden Substanzen, oberflächenaktiven Mitteln, Konservierungsmitteln, Entschäumern, Fetten, Ölen, Wachsen, Bakteriziden, Parfümen, Treibmitteln, Farbstoffen und/oder Pigmenten oder dergleichen ausgewählt sind.

7. Verwendung nach Anspruch 1, zur Herstellung einer oral zu verabreichenden Zusammensetzung.

8. Verwendung nach Anspruch 7,
**dadurch gekennzeichnet, dass** die Antioxidantien ausgewählt sind aus der Gruppe, bestehend aus Vitamin E und dessen Derivate, Vitamin C und dessen Derivate, Beta-Carotin, Lykopin und dessen Derivate, Lutein und dessen Derivate, Troxerutin und dessen Derivate, Rutin und dessen Derivate ausgewählt sind.

9. Verwendung nach Anspruch 7 oder 8,
**dadurch gekennzeichnet, dass** der Gehalt an Antioxidantien in der Zusammensetzung 1-600 mg beträgt, bevorzugt 2-300 mg, besonders bevorzugt 5-200 mg.

10. Verwendung nach einem der Ansprüche 7 bis 9,
**dadurch gekennzeichnet, dass** die Zusammensetzung des weiteren Hilfsstoffe enthält, die unter Lösungsmitteln, Lösungsbeschleunigern, Emulgatoren, Lösungsvermittlern, Netzmitteln, Antischaummitteln, Salzbildnern, Puffern, Gelbildnern, Verdickungsmitteln, Filmbildnern, Gleitmitteln, Schmiermitteln, Formtrennmitteln, Fließregulierungsmitteln, Zerfallsbeschleunigern oder Sprengmitteln, Sorptionsmitteln, Füllmitteln, Konservierungsmitteln, Geschmacks- und Geruchskorrigenzien sowie Färbemitteln ausgewählt sind.

11. Pharmazeutische oder kosmetische Zusammensetzung, umfassend einen wirksamen Gehalt an Antioxidantien zum Schutz der Haut vor IR-Strahlung.
